# EUROPEAN PATENT APPLICATION

(11) **EP 4 338 655 A1**
(43) Date of publication of application: **20.03.2024**
(21) Application number: 22196223.6
(22) Date of filing: 16.09.2022
(51) Int. Cl.: A61B 3/00, A61F 2/16

(54) **CUSTOMIZING INTRAOCULAR LENSES**

(71) Applicant: Ligi Tecnologie Medicali S.r.l., 74100 Taranto (IT)
(72) Inventor: D'IPPOLITO, Giuseppe, 74121 Taranto (IT)
(74) Representative: Bardehle Pagenberg Partnerschaft mbB Patentanwälte Rechtsanwälte

(57) **Abstract**

An apparatus may be provided for determining at least one parameter of a lens (150) for an eye (100), the apparatus comprising: a. means for obtaining topographic information of an anterior surface (110) and topographic information of a posterior surface (120) of a cornea of the eye (100); b. means for obtaining distance information (160) of a retina (190) of the eye (100) relative to the anterior surface (110) of the cornea of the eye (100); c. means for determining the at least one parameter of the lens (150) of the eye (100) such as to optimize focusing on the retina (190) by ray tracing at least in part based on the topographic information of the anterior surface (110) of the cornea, the topographic information of the posterior surface (120) of the cornea and the distance information (160). A corresponding method and computer program may be provided as well.

## Description

### 1. Technical field:

The present invention relates to determining at least one parameter of a lens for an eye, such as an implantable intra-ocular lens and a corresponding apparatus, a method and computer program.

### 2. Description of the prior art:

Cataract surgery is the most prevalent eye surgery. Many patients develop a cataract in their eye(s) when getting older which is characterized by an opacification of the natural lens of the eye(s). During cataract surgery the opaque lens is surgically removed, and a variety of surgical techniques have been developed. Typically, an artificial lens is inserted to replace the natural lens, and this artificial lens is also generally referred to as intraocular lens (IOL).

It is general practice that the IOL is selected based on parameters such as spherical diopter value, and possibly also on a cylindrical diopter value and a cylinder axis, to optimize the vision of the eye of the patient. To this end, several regression models are typically used to determine the parameters of the IOL to be selected (for example a recommended spherical diopter value of the lens, a cylindrical diopter value and a cylinder axis of the lens).

However, the regression models are only statistical models that provide a recommendation of those lens parameters that, according to the selected regression model constitute the statistical optimum for eyes having the same or similar parameters (e.g. that statistically resulted in the best vision for eyes having the same or similar parameters). Hence, the known regression models are typically less than optimal. A relatively large fraction of patients thus still needs glasses after IOL implantation. As a result, that there is a need for improving the selection of lenses for eyes.

### 3. Summary of the invention:

According to an aspect, the above need is at least partly met by an apparatus according to claim 1, and by a corresponding computer method and a corresponding computer program according to claims 11 and 15, respectively.

An apparatus for determining at least one parameter of a lens for an eye may be provided. The apparatus may comprise means for obtaining topographic information of an anterior surface and topographic information of a posterior surface of a cornea of the eye. It may further comprise means for obtaining distance information of a retina of the eye relative to the anterior surface of the cornea of the eye. Further the apparatus may comprise means for determining the at least one parameter of the lens of the eye such as to optimize focusing on the retina by ray tracing at least in part based on the topographic information of the anterior surface of the cornea, the topographic information of the posterior surface of the cornea and the distance information .

For example, by the ray tracing, one or more light rays may be considered that are refracted according to the topographic information of the anterior surface and of the posterior surface of the cornea, and by the lens of the eye according to the at least one parameter of the lens. The ray(s) may then impinge on the retina, which may be located at a distance from the anterior surface of the cornea defined by the distance information. The means for determining may vary the at least one parameter of the lens such as to optimize focusing of the light ray(s) onto the retina, e.g. its center (e.g. as defined by the fovea and/or the optical axis of the eye).

Hence, the at least one parameter of the lens may be tailored to the individual eye, instead of being an average recommendation based on statistically sampling over a multitude of eyes, such that an optimum lens (e.g. IOL) may be determined for the eye. Particularly, such multitude of eyes may differ in various aspects which are not reflected in the regression model, which may indeed be considered according to the present invention. This specifically applies to possible irregular shapes of anterior and/or posterior corneal surfaces of the cornea. While a first IOL maybe ideal for an eye having a certain average corneal power (e.g. as expressed by a spherical diopter value), it may be less than ideal for another eye having the same certain average corneal power, due to the different topographies of anterior and/or posterior corneal surfaces. These drawbacks may be avoided by the present invention such that IOL selection may be improved leading to better vision of each individual patient after IOL implantation (e.g. in the context of a cataract surgery).

For example, the at least one parameter of the lens may include at least one of: a spherical refractive power of the lens, a cylindrical refractive power of the lens, a cylinder axis of the lens, an asphericity value of the lens. In some examples, the at least one parameter may additionally or alternatively include one or more parameters that define a customized profile of the lens to compensate at least one or more higher order aberrations of the eye (i.e. corresponding to Zernike polynomials that go beyond vision correction by spherical diopter values and/or astigmatism correction). For example, the one or more parameters may relate to coefficients corresponding to Zernike polynomials in general. All these parameters may thus be optimally selected for the lens for the eye.

In some examples, additionally or alternatively the at least one parameter may include a thickness of the lens, a position of the lens, a refractive index of the lens, a type of lens, a profile of the lens, a diameter of lens.

For example, while the position of the lens will coarsely be predetermined for the eye by its anterior chamber length, lenses implanted may ultimately position themselves at slightly different positions compared to the (pre-operative) anterior chamber depth. Hence, the position may be a parameter that can be adjusted according to a (ideal) position as determined by the optimization as outlined herein, by selecting corresponding IOLs.

The type of the lens may for example be: sphere, toroid, conoid or aconic. In some examples, the type may be selected from a group consisting of: sphere, toroid, conoid or aconic. In some examples, the group may be complemented by further geometries (with and/or without asphericity) that define surfaces including components for further, higher order compensation than those required for describing spherical diopter values and astigmatism correction. Thus would allow the customization of lenses that also correct higher order aberrations (e.g. higher than spherical refraction and astigmatism).

The profile of the lens may for example be: symmetrical biconvex or plano-convex. In some examples, the type may be selected from a group consisting of: symmetrical biconvex and plano-convex sphere. In some examples, the group may be complemented by descriptions of a typical lens surface (e.g. concave, etc.).

In some examples, the means for determining may be adapted to optimize a quantity representative of focus quality of rays impinging on the retina. Hence, the focus quality maybe optimized by determining the at least one parameter of the lens accordingly.

In some examples, the quantity representative of focus quality may be a distance of the position(s) of the ray-traced beams refracted, through the anterior and posterior corneal surfaces, impinging the retina, from a center of the retina (e.g. as defined by a fovea and/or an optical axis of the eye) and this quantity may be minimized. For example, a position of intersection with the retina (e.g. modelled as a plane and/or a curved surface) may be determined for each of the one or more rays, and a distance to the center of the retina may be determined for each ray. A mean value thereof (e.g. a root-mean-square value) may be used as the distance. But it is also conceivable to determine a mean focusing point for the one or more rays, e.g. defined by the center of gravity of their points of intersection with the optical axis, and then minimize the distance of this mean focusing point to the retina.

Another exemplary quantity is a point spread function (PSF) of rays impinging on the retina. The means for determining may for example be adapted to minimize an error of the point spread function (e.g. an RMS deviation from an ideal point) of rays impinging on the retina. The rays used in the ray tracing will generate a certain illuminated pattern on the retina (point spread function). By minimizing the deviation of this pattern from an ideal point (e.g. at a center of the retina; e.g. at an optical axis of the eye, e.g. corresponding to the anterior and posterior corneal surfaces, e.g. as defined by an apex of the cornea), it may be ensured that the light rays are optimally focused onto the retina.

Another possible option may be to maximize a modulation transfer function (MTF) of the eye. A MTF pertains to the contrast and resolution of a lens from the center to its edges against a "perfect" lens that would transmit 100% of the light that passes through it. For example, at least a portion of an MTF chart could be generated using ray tracing. The means for determining may thus for example be adapted to maximize the MTF (chart).

In some examples, a metric may be optimized, e.g. by the means for determining, that comprises two or more of the above quantities.

The apparatus may further comprise means for obtaining at least one predetermined property of the lens and the means for determining are adapted such as to optimize focusing on the retina by ray tracing at least in part based on the predetermined property. In general, it is possible that a plurality of lens parameters are determined by the means for determining to optimize the design of the lens for the eye. In practice, however, it may be helpful to predetermine at least one property of the lens. This property may then be fixed, whereas other lens parameters may be varied during ray tracing such as to optimize focusing, as outlined herein. For example, a set of properties of the lens may be predetermined and the remaining parameters may be determined by the means for determining based on optimizing focusing in the ray tracing process.

The at least one predetermined property of the lens may comprise at least one of: a position of the lens, a refractive index of the lens, a (central) thickness of the lens, a type of lens, a diameter of the lens, a profile of the lens. The apparatus may comprise means for obtaining these properties. For example, the position of the lens may be predetermined by an anterior chamber depth of the eye (pre-operative). Additionally, deviations from the anterior chamber depth may be taken into account, such as potential position shifts that are expected for certain IOLs (in fact, manufacturers of IOLs typically publish such position shifts). In short, the position of the lens may include an expected position of a specific IOL (e.g. according to the specifications of the manufacturer) after implantation. This position may be relative to the position of a typical anterior chamber length. Additionally or alternatively, it may be used relative to a specific anterior chamber length as acquired for the specific eye (e.g. by a biometer, that may or may not be part of the apparatus outlined herein).

Hence, for example, various lens parameters may be predetermined, and only the remaining parameters may be optimized as outlined herein.

Topographic information of a surface of the cornea (e.g., anterior and/or posterior surface) may include a profile (e.g., a plurality of coordinates defining one or more actual points on the surface of the cornea, a plurality of points approximating the surface of the cornea, or a function fitted to the surface of the cornea, etc.). The topographic information may for example be provided in the form of a number of points in an x-y-z coordinate system. Of course, other coordinate systems may be used.

It is noted that the apparatus described above may not necessarily include means for determining or calculating the topographic information and/or the distance information (or any of the predetermined properties of the lens). The means for obtaining of the apparatus (that may also be implemented as means for obtaining any of the predetermined properties of the lens) may be implemented by means for receiving the respective information from other devices or an operator of the apparatus. In such examples, the apparatus may use the received information directly for using it in the ray tracing process, and/or it may further process the received information. For example, the topographic information and/or the distance information (or any of the predetermined properties) relating to a patient maybe retrieved from a memory, a database, a server in a cloud, and/or from one or more corresponding diagnostic devices (e.g. a corneal tomographer/topographer for the topographic information and/or a stromal interface and/or an entrance pupil; a biometer, an optical coherence tomography device, etc. for an axial length of the eye, and/or an anterior chamber length, etc.), or these may simply be input by an operator of the apparatus, e.g., via a corresponding (graphical) user interface.

In turn, the at least one parameter of the lens may for example be stored by the apparatus, sent to another device, and/or output to the operator of the apparatus, e.g. via a corresponding (graphical) user interface.

But in some examples, the means for obtaining the topographic information may comprise means for measuring the topography of the anterior and/or posterior surfaces of the cornea and thus provide the topographic information of the anterior and/or posterior surfaces. Additionally or alternatively, the means for obtaining the distance information may comprise means for measuring a distance, e.g. an axial length of the eye, and thus provide the distance information. Additionally or alternatively, the apparatus may comprise means for measuring and/or determining topographic information of the stromal interface, of the epithelium thickness, of the stromal thickness, of the corneal thickness (e.g. pachymetry), an entrance pupil position, an entrance pupil diameter (e.g. by a corneal tomographer), an anterior chamber depth, and/or a lens thickness (e.g. by a biometer, an optical coherence tomographer, etc.), for example.

In some examples, the topographic information of the anterior surface and the topographic information of the posterior surface of the cornea each comprises a plurality of data points, e.g. at least two data points, at least three data points, at least five data points, at least ten data points, etc., spanning at least a surface area of 100 µm² on the anterior surface as well as the posterior surface. For example, the data points maybe arranged on a (regular) grid spanning the surface area. In other examples, the surface area may comprise at least 500 µm², at least 1000 µm² or at least 2000 µm². The area may preferably include a center of the cornea, e.g. as defined by an apex of the cornea, and/or it may be centered around the center of the cornea.

In some examples, at least 10, at least 50, or at least 100 light rays are used for optimizing focusing on the retina by ray tracing based on the topographic information of the anterior surface of the cornea and the topographic information of the posterior surface of the cornea.

In some examples, the apparatus may comprise means for obtaining at least one parameter of an entrance pupil of the eye. The means for determining may be adapted such as to optimize focusing on the retina by ray tracing at least in part based on the at least one parameter of the entrance pupil of the eye. Hence, effects of entrance pupils of individual eyes may be taken into account when optimizing the lens.

The at least one parameter of the entrance pupil may comprise a distance of the entrance pupil, e.g. relative to the anterior surface of the cornea and/or a diameter of the entrance pupil.

In some examples, the apparatus may comprise means for obtaining topographic information of a stromal interface of the cornea. The means for determining may be adapted such as to optimize focusing on the retina by ray tracing at least in part based on the topographic information of the stromal interface of the cornea. Hence, also the refraction at the stromal interface of the individual eye may be taken into account when determining the at least one parameter of the lens, such that optimization of the lens for the individual eye may be even further improved.

In some examples, the apparatus may comprise means for obtaining an anterior chamber depth of the eye. The means for determining may be adapted such as to optimize focusing on the retina by ray tracing at least in part based on the anterior chamber depth of the eye. Hence, the position of the lens specific for the individual eye may be taken into account when determining the at least one parameter of the lens. During the optimization process, an expected position deviation from the specific anterior chamber depth of the eye may be taken into account, which may be specific to the particular lens used.

The lens may be an intra-ocular lens to be implanted into the eye. Hence, the patient vision after implantation may be improved.

Another example relates to a computer-implemented method for determining at least one parameter of a lens for an eye. The method may comprise the following steps: Topographic information of an anterior surface and topographic information of a posterior surface of a cornea of the eye may be obtained. Distance information of a retina of the eye relative to the anterior surface of the cornea of the eye may be obtained. At least one parameter of the lens of the eye may be determined such as to optimize focusing on the retina by ray tracing at least in part based on the topographic information of the anterior surface of the cornea, the topographic information of the posterior surface of the cornea and the distance information.

The at least one parameter may include at least one of: a spherical refractive power of the lens, a cylindrical refractive power of the lens, a cylinder axis of the lens, an asphericity value of the lens, a customized profile of the lens to compensate at least a higher order aberration of the eye.

The determining may include optimizing a quantity representative of focus quality of rays impinging on the retina.

The method may further comprise obtaining at least one predetermined property of the lens. The determining may include optimizing focusing on the retina by ray tracing at least in part based on the predetermined property.

The method may further comprise selecting an intraocular lens to be implanted into the eye at least in part based on the at least one determined parameter of the lens.

Further, a computer program may be provided for determining at least one parameter of a lens of an eye. The computer program may comprise instructions which, when executed by a computer, cause the computer to implement the following steps: Obtaining topographic information of an anterior surface and topographic information of a posterior surface of a cornea of the eye. Obtaining distance information of a retina of the eye relative to the anterior surface of the cornea of the eye. Determining the at least one parameter of the lens of the eye such as to optimize focusing on the retina by ray tracing at least in part based on the topographic information of the anterior surface of the cornea, the topographic information of the posterior surface of the cornea and the distance information.

It is noted that all aspects described herein may be implemented as a corresponding functionality (means) of the apparatus described herein, as a corresponding step of the methods outlined herein and/or as a corresponding instruction of the computer programs outlined herein. Even if described with reference to an apparatus, method and/or computer program, the aspects outlined herein may be applied to the respective other of an apparatus, method and/or computer program.

### 4. Brief description of the Figures:

Possible embodiments of the present invention will be described in more detail in the subsequent detailed description with reference to the following Figures:
- Figs. 1A, 1B:: Examples for raytracing based on topographic information of the anterior and posterior surfaces of the cornea and based on distance information of the retina (e.g. relative to the anterior surface of the cornea) leading to a non-optimal focusing point (Fig. 1A) and to an optimal focusing point (Fig. 1B);
- Figs. 2A-F: First example for a regular astigmatic corneal surface for which a lens optimized by ray tracing may improve vision.
- Figs. 3A-E: Second example for an irregular corneal surface for which a lens optimized by ray tracing may improve vision.
- Fig. 4:: Further example for raytracing based on topographic information of the anterior and posterior surfaces of the cornea and based on distance information of the retina.
- Figs. 5A-E: Examples for further optimizing vision of the eye comprising the irregular corneal surface of Figs. 3A-E.

### 5. Detailed description of possible embodiments:

Possible embodiments of the present invention will be described in the following. For brevity, only a few embodiments can be described. The skilled person will recognize that the specific features described with reference to these embodiments may be modified and combined differently and that individual features may also be omitted if they are not essential. The general explanations in the sections above will also be valid for the following more detailed explanations.

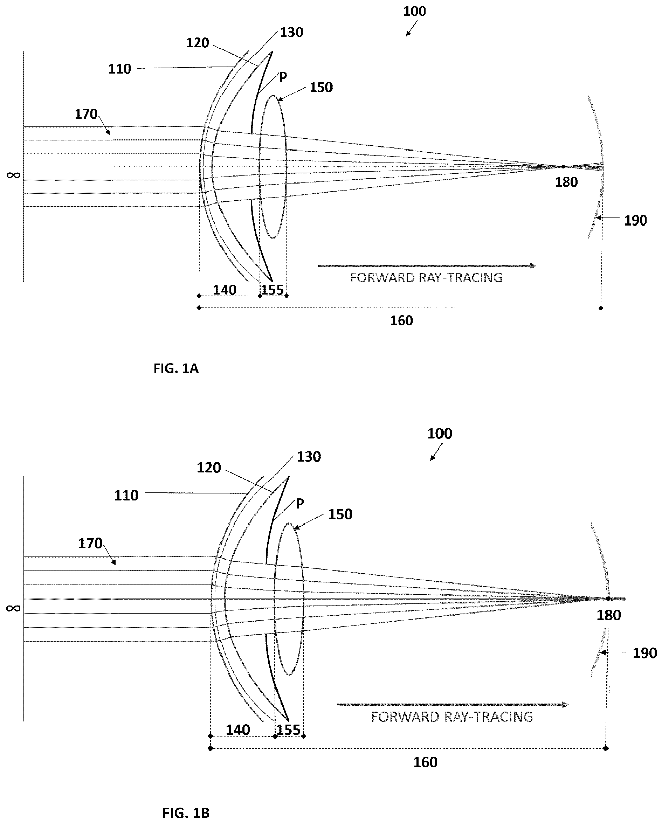

Fig. 1 shows an exemplary ray tracing situation in an eye model 100. In this example a plurality of light rays 170 are used. These are arranged parallel around an optical axis of eye 100, such as to define a situation of far vision. The rays may be arranged on a regular grid center and arranged symmetrically around the optical axis of eye 100 (not shown). In other examples, also different rays may be used, e.g., in case near-vision and/or intermediate-distance vision is to be optimized.

Rays 170 propagate from left to right and impinge on the anterior corneal surface 110. There, they are refracted according to Snell's law. This refraction may be calculated for each ray 170 based on the topographic information of the anterior corneal surface 110.

Rays 170 then further propagate within the cornea, being refracted again at the stromal interface 130 of the cornea. The refraction at this interface may optionally be calculated based on the topographic information of the stromal interface 130. In other examples, this interface and the refraction at this interface may be ignored.

The rays 170 then impinge on the posterior corneal surface 120 and are refracted again. This refraction maybe calculated based on the topographic information of the posterior corneal surface 120.

The rays 170 then, optionally in the ray tracing process, pass an entrance pupil P which may block one or more of the rays 170. Whether or not a ray 170 is blocked may be determined based on the at least one parameter of the entrance pupil (e.g. its position and/or diameter).

The rays 170 passing through entrance pupil P then impinge on lens 150 with its anterior surface positioned at a distance 140 (anterior chamber depth) from the anterior corneal surface. Rays 170 are refracted by the lens 150 having a certain lens thickness 155 and then impinge on the retina 190 positioned at distance 160, e.g. with respect to the anterior corneal surface 120.

The refractive values of the lens may be calculated based on the at least one parameter of the lens to be determined. Additionally, one or more predetermined property of the individual lens may be used in the calculation. For example, a type of the lens, a central thickness of the lens, an expected position of the lens (relative to the anterior chamber depth 140), a refractive index of the lens, a profile of the lens, and/or a diameter of the lens may be used.

As outlined herein, the type of the lens may for example be: sphere, toroid, conoid or aconic. In some examples, the type may be selected from a group consisting of: sphere, toroid, conoid or aconic. If a sphere is used, this implies that only a spherical diopter value is used when calculating the refraction at the lens. If a toroid is used, this implies that not only a spherical diopter value is used, but also a cylindrical diopter value and a cylinder axis. If a conoid (or aconic) lens type is used, this implies that in addition to a spherical diopter value (and a cylindrical diopter value and a cylinder axis) an asphericity value of the lens is used. The lens type may also be customized to correct the higher orders of aberrations of the eye.

It is noted that at least the topographic information of the anterior corneal surface 110, the topographic information of the posterior corneal surface 120, and the information on the distance 160 may be specific to the individual eye (and corresponding information may be obtained by an apparatus as outlined herein). The at least one predetermined property may be specific to the individual lens (e.g. to a specific IOL that is to be implanted and whose further parameters are to be optimized). For the further parameters, such as relating to anterior chamber depth 140, refractive index of the cornea, for example, standard parameters may be used, but it is also within the scope of the present application to use customized parameters for these as well. The same applies to further parameters such as the stromal interface 130 and the pupil if these are used in the ray tracing process.

The at least one parameter of the lens may be varied (e.g. systematically) in the ray tracing process, such that the focusing point 180 of the rays is not offset from the position of the retina 190 as shown in Fig. 1A, but that the focusing point 180 is positioned on the retina 190, e.g. at the center of the retina (e.g. as defined by the fovea and/or the optical axis of eye 100).

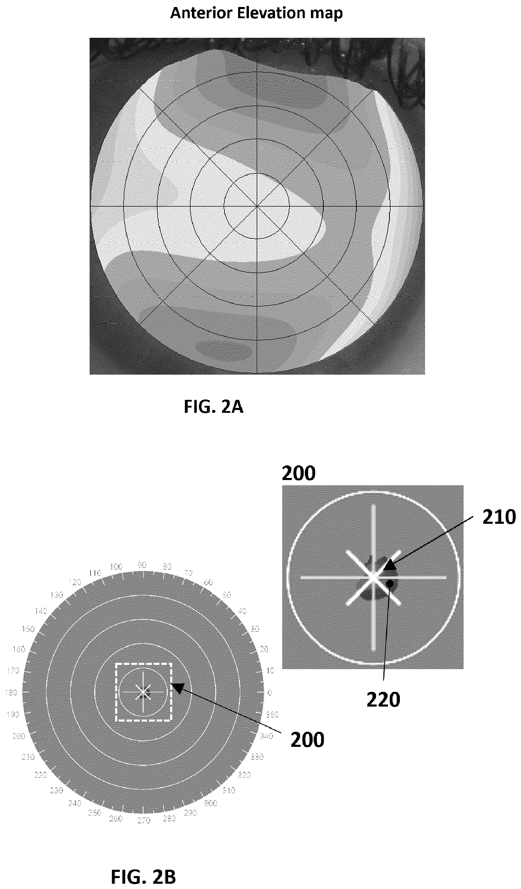

Figs. 2A-F show various aspects related to a first example of an eye.

Fig. 2A shows topographic information of an anterior surface of an eye. As can be discerned the topography of the anterior surface includes an astigmatic contribution. By performing ray tracing based on the topographic information of Fig. 2A as outlined herein, e.g. with reference to Figs. 1A and 1B, the positions of rays, refracted by the anterior surface of the eye (and by the posterior surface of the eye based on corresponding topographic information which is not shown) and a lens, and subsequently impinging on the retina (as defined by the distance information) may be determined. One or more parameters of the lens (relevant for refraction) may be varied such as to optimize focusing on (the center of) the retina. To this end, various algorithms may be used.

Fig. 2B shows the positions of rays on the retina, represented by a circular plane, after optimization. In this example, a toric lens was assumed, i.e. the parameters of the lens that were determined/optimized were: spherical diopter value, cylindrical diopter value, cylinder axis. In this specific example, the determined values were: 16.92 D (sphere), 2.06 D (cylinder), 74° (cylinder axis). The determination was additionally based on the predetermined properties of a lens refractive index of 1.5, a lens diameter of 6 mm, a symmetrical biconvex lens profile, and a toroidal lens type. Of course, these are only examples, and for other topographies of the cornea, different distance information, and/or different predetermined parameters of the lens different results may be obtained.

A zoom into a central portion 200 of the retina is separately shown. The rays on the retina are indicated by reference sign 220. As can be seen, they are all closely located at and around the center 210 of the retina.

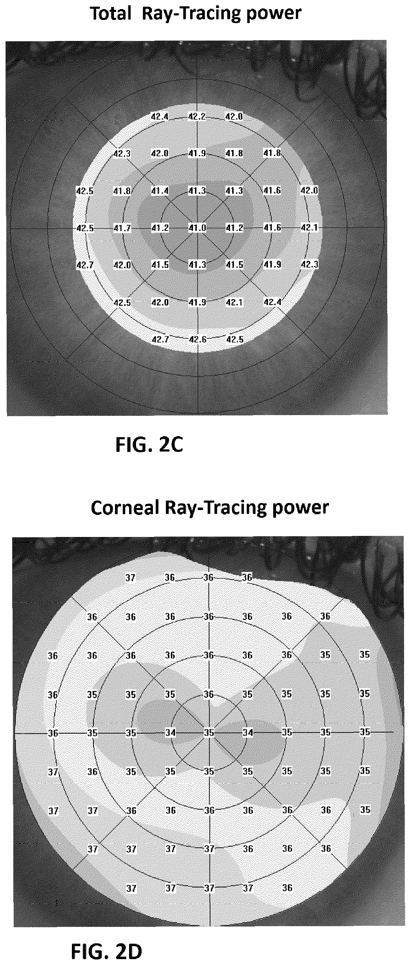

Fig. 2C shows the total (ray-tracing) refractive power of the eye, as a function of the corneal data, which was obtained with the mentioned optimized lens parameters. The total refractive power is uniform across the entire eye and no relevant astigmatism is visible. Moreover, the absolute values are about 41.0 D (center), 41.2 to 41.5 D in a periphery around the center, and across the entire cornea (within an entrance pupil defined by the pupil) the values still remain within an upper bound of 42.7. Hence, excellent vision can be expected for this eye. The variation of the refractive power across the cornea thus varies within about 4% or less.

Fig. 2D shows the result that would be obtained for the eye without any lens, i.e., the refractive contribution of the cornea, only. As can be seen, the result in Fig. 2C shows astigmatism and generally a lower refractive power due to the lack of the lens (only about 35 D at the center). The refractive power varies between 34 and 37 in this example, i.e. almost 10%, across the cornea. Most importantly, without compensation by the optimized lens, the astigmatism would be present. With the optimized lens, this astigmatism can be corrected (cf. Fig. 2C).

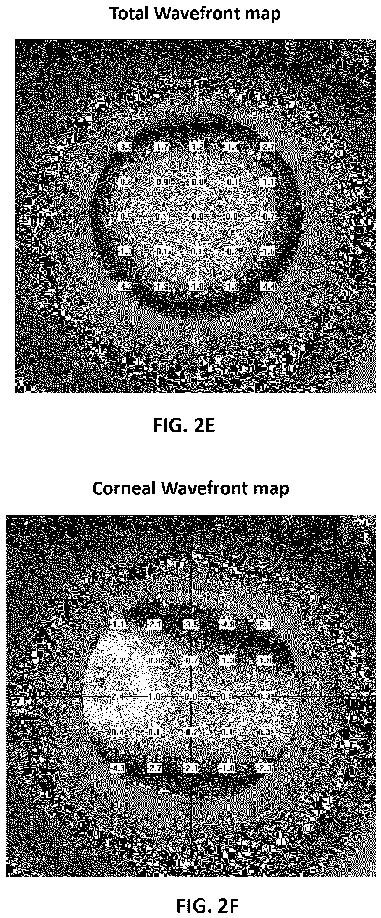

That the vision is close to ideal with the optimized lens can also be seen from Fig. 2E. It shows a wavefront map of the eye across the surface of the cornea including the contribution of the lens. As can be seen, the deviations from the zero point at the center of the cornea are small. There is an extended area at the center in which the deviations are at most ±0.2 µm. This area may have a diameter equal to the entrance pupil, for example.

For comparison, a map of the wavefront is shown in Fig. 2F that would be obtained without any lens. Clearly, strong deviations above ±1 µm are present in the extended area at the center which can be contained to within ±0.2 using the optimized lens. Also the pronounced astigmatism visible in Fig. 2F is compensated by the optimized lens as shown in Fig. 2E.

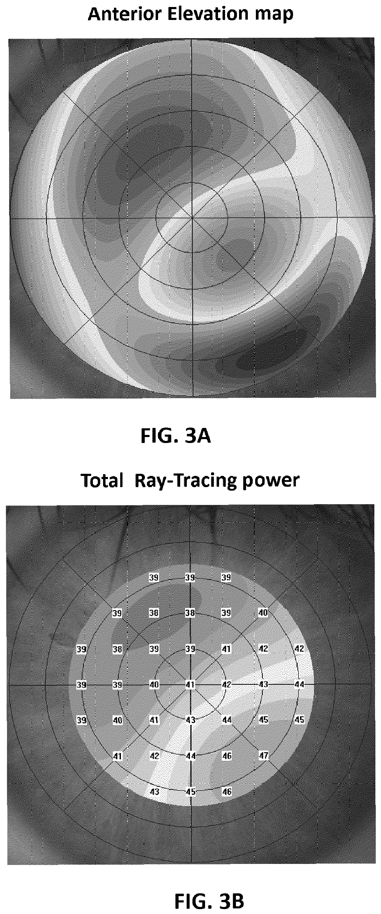

Figs. 3A-E show various aspects related to a second example of an eye. In contrast to the cornea in the example of Figs. 2A-F, the cornea used in the second example comprises an irregular disorder, namely an ectatic disorder in the form of a keratoconus which leads to higher order aberrations caused by the cornea.

Fig. 3A shows topographic information of an anterior surface of an eye. As can be discerned, the topography of the anterior surface is irregular. Similarly as described with reference to Fig. 2A, a toric lens was optimized for that anterior surface (and a corresponding posterior surface and a corresponding axial length of the eye, both are not shown). In this example (for predetermined properties of the lens as outlined in the example of Figs. 2A-F), a spherical diopter value of 11.74 D, a cylindrical diopter value of 2.56 and a cylinder axis of 54° was obtained.

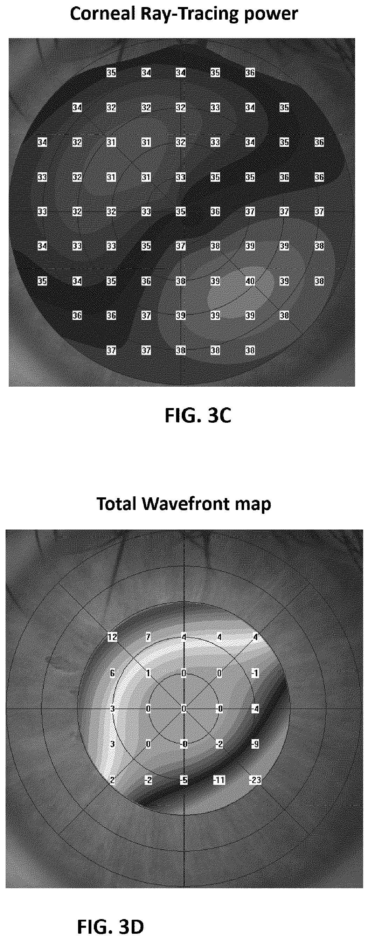

Figs. 3B show the total refractive power with the optimized lens (Fig. 3B) and without any lens (Fig. 3C). As can be seen from Fig. 3B, the total refractive power of the eye is brought into the desired range (about 41 D at the center). Moreover, the variation of the refractive power across the cornea is within 38 and 47 which is a variation of less than 10%. Fig. 3C shows the result without any lens. Here the refraction is lower, as expected. But also the variation of the refraction across the cornea is increased. For example, it varies from 31 to 40, which is above 10%.

However, still with the toric lens that was used for optimization, as can be seen from Fig. 3B, the influence of the keratoconus cannot be fully eliminated. Hence, the vision for this eye is not expected to be perfect using a toric lens, despite the optimization. However, as outlined herein, also a lens may be used with more degrees of freedom, e.g. for correcting higher order aberrations. Generally, using such a lens may allow to further optimize vision, e.g. eliminate the higher order aberration effects of the keratoconus in this example.

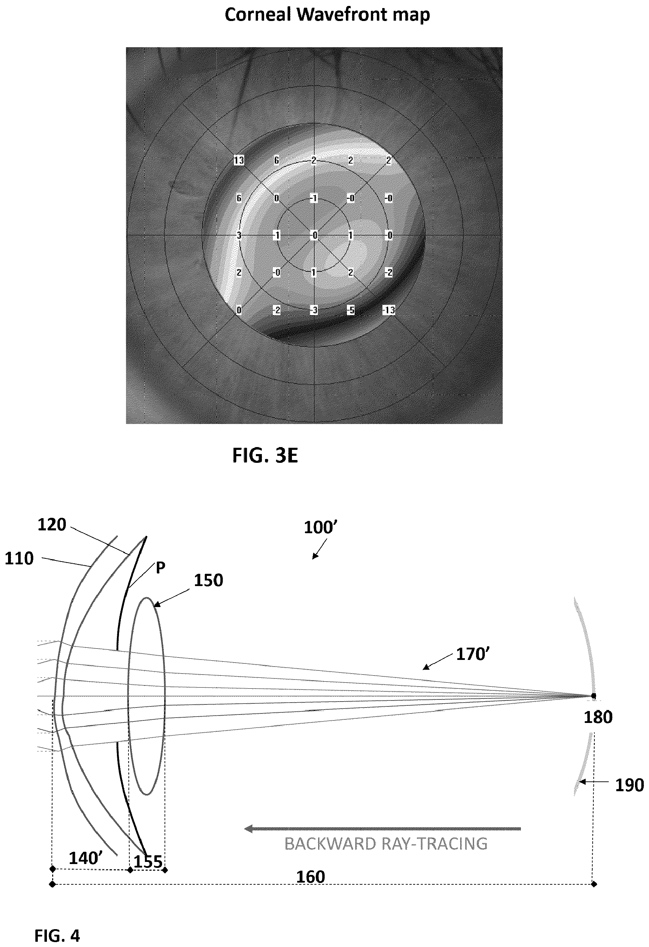

Figs. 3D and 3E show wavefront maps of the eye with the optimized lens (Fig. 3D) and without any lens (Fig. 3E). As can be seen, the vision in the center of the eye can be notably improved. For example, the RMS deviation from zero in the eight measured points around the center is 0.625 with optimized lens and 1 for the eye without lens. However, at the outer periphery of the cornea, the aberration cannot be fully corrected due to the limitation to a toric lens. These aberrations may be corrected by using a lens with further degrees of freedom for also compensating higher order aberration and/or additional corneal refractive surgery, as described below.

An alternative to using a lens with degrees of freedom to correct higher order aberrations, would be to correct the remaining higher order aberrations by corneal refractive surgery (e.g. using laser ablation). In some examples, the spherical refraction (and optionally the cylindrical refraction) of the eye may be optimized, eventually using a laser ablation profile based on the effective anterior chamber depth of the IOL, after the implantation, e.g. as measured for example with a biometer. The remaining higher order aberration (and optionally the remaining spherical and cylindrical aberration) may then be output. For example, data defining the remaining aberrations and/or the ablation profile may be output, such that it may be used by a laser ablation apparatus for ablating corneal tissue.

Fig. 4 shows a further ray-tracing process that maybe carried out subsequent to the aspects described above. All reference signs shown in Fig. 4 that are identical to those of Fig. 1 may designate the same elements.

In Fig. 4, the ray-tracing process employs rays 170' emerging from the focusing point 180 towards the posterior and anterior surfaces 120, 110 of the eye model 100'.

Before impinging on these surfaces of the cornea, they are refracted by the lens 150. However, Fig. 4 may relate to a post-operative situation, wherein a presently not easily controllable shift of the position of the lens 150 in form of an IOL in axial direction may occur. For example, the position of IOL 150 in the patient may be slightly different to that planned. This may be manifested in a slightly different anterior chamber depth 140' which may be measured for the individual eye post-operatively, e.g. as outlined above. Due to this potentially different anterior chamber depth 140' which may be measured post-operatively, the vision of the patient may not be optimal.

Another reason, why the vision may not be optimal may be given by the limitation of the lens model, as outlined above. For example, a lens 150 may be used as an IOL that only corrects spherical refractive power (and possibly cylindrical refractive power).

Therefore, it is a further aspect of the present invention to determine a (post-operative and/or remaining) aberration of an eye by a ray-tracing process. The ray-tracing process maybe backwards as shown in the example of Fig. 4. In this aspect, the anterior surface 110 of the cornea may be modified (e.g. simulating an ablation) such as to optimize refraction of the eye, e.g. by minimizing the RMS angle deviations of the rays emerging from the cornea from the optical axis (horizontal line in Fig. 4). In other words, that modified anterior surface 110 (or that ablation profile) may be determined that optimizes refraction, at least in part based on parameters characterizing the lens 150 (e.g. as outlined herein), topographic information of the posterior corneal surface 120 and topographic information of the modified anterior corneal surface 110. Also, the distance information 160 as outlined herein may be used therein, e.g. similarly as outlined with reference to Fig. 1. Optionally, the post-operatively measured anterior chamber depth 140' may be used in the optimization process, instead of the (expected) anterior chamber depth 140. But in other examples, the same anterior chamber depths may be used, e.g. in which case still higher order aberrations, not compensated by the IOL 150, may be compensated by refractive surgery. Optionally, also further parameters may be used, e.g. as outlined herein, such as parameters specific to the entrance pupil P, etc.

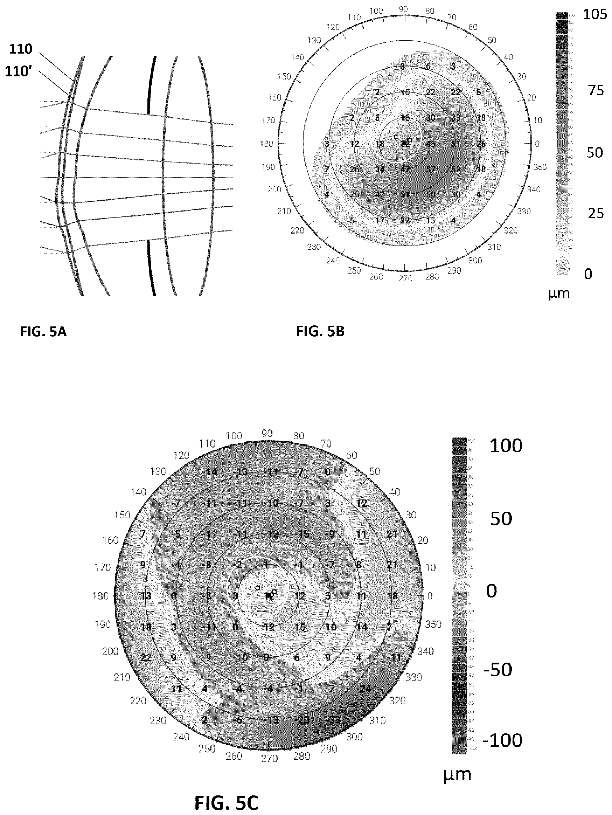

Fig. 5A shows a zoom into the relevant portion of the cornea contributing to the refraction shown in Fig. 4. The rays impinging on the anterior corneal surface 110 (indicated with solid lines) generally exit the cornea with an angle deviation from the optical axis (horizontal line in Fig. 5A). By ablating a portion of the cornea such as to realize modified anterior corneal surface 110', all rays refracted at that modified corneal surface 110' (indicated by dashed lines) exit the cornea essentially along the optical axis, such that vision is optimized.

An exemplary ablation profile is shown in Fig. 5B. Notably, the ablation profile in Fig. 5B corresponds to that necessary to correct the remaining aberrations of the eye shown in Figs. 3A-E post-operatively, i.e. after insertion of the optimized lens as an IOL.

Fig. 5C shows the optimized corneal anterior profile (after ablation). With this profile, vision of the patient can be optimized.

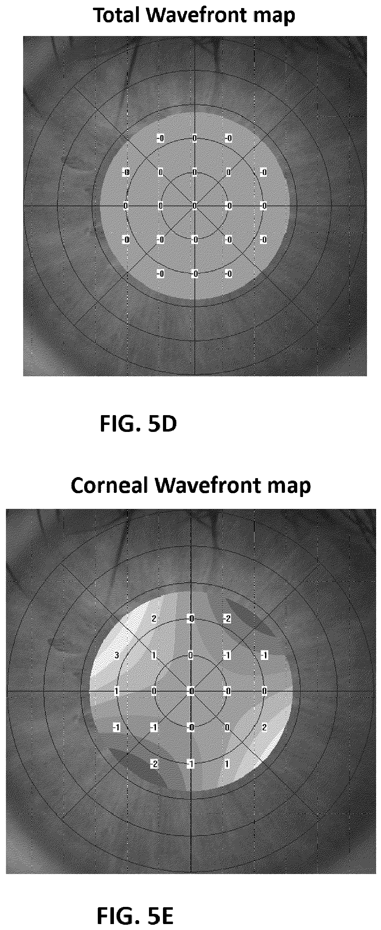

This is further shown in Fig. 5D which shows a wavefront map of the eye across the surface of the cornea including the contribution of the optimized IOL and the optimized corneal anterior profile. As can be seen, there is no measurable deviation from zero (in units of µm) across the entire cornea.

Fig. 5E shows the wavefront map for the cornea (i.e. without the lens contribution; unit: µm). As can be seen, the cornea is astigmatic. This is based on the prior optimization of the IOL that was optimized such as to compensate the astigmatism of the cornea. Hence, in the subsequent optimization of the cornea, this astigmatism did not need to be corrected anymore.

In summary, by the aspects outlined herein with respect to determining at least one parameter of a lens, all parameters of lenses for eyes (e.g. IOLs) may be optimized. If desirable, only a spherical refraction error (and optionally a cylindrical refraction error) may be minimized by the lens. Remaining higher order aberrations may then be output as input for a laser ablation apparatus, such that it may be compensated by refractive surgery. Additionally or alternatively, deviations of the IOL position after IOL surgery from the expected position may be determined such that any remaining aberrations (that may also include a spherical refraction error as well as a cylindrical refraction error) may be determined accordingly and output in the form of data that can be used by a laser ablation apparatus or e.g. directly in the form of an ablation profile that may be read by a laser ablation apparatus.

The apparatus outlined herein may generally further comprise means for determining a residual aberration of the eye comprising the IOL having the at least one (optimized) parameter. Hence, the residual aberration after IOL optimization maybe determined. This may optionally include taking into account the post-operative position of the IOL within the eye.

Additionally or alternatively, the apparatus outlined herein may generally further comprise means for determining an optimized corneal anterior surface and/or a corneal ablation volume for correcting a residual aberration of the eye comprising the lens having the at least one parameter. Hence, the apparatus may output, e.g. via a user interface, via a wired and/or wireless connection, etc. data that may be used by a laser ablation apparatus to perform a refractive surgery to eliminate the residual ablation. In some examples, the apparatus outlined herein may comprise means for performing the laser ablation.

It is emphasized that the aspects outlined hereinbefore with respect to an apparatus may be provided as features or instructions of a computer program and/or as methods steps.

## Claims

1. An apparatus for determining at least one parameter of a lens (150) for an eye (100), the apparatus comprising:
a. means for obtaining topographic information of an anterior surface (110) and topographic information of a posterior surface (120) of a cornea of the eye (100);
b. means for obtaining distance information (160) of a retina (190) of the eye (100) relative to the anterior surface (110) of the cornea of the eye (100);
c. means for determining the at least one parameter of the lens (150) of the eye (100) such as to optimize focusing on the retina (190) by ray tracing at least in part based on the topographic information of the anterior surface (110) of the cornea, the topographic information of the posterior surface (120) of the cornea and the distance information (160).

2. The apparatus according to claim 1, wherein the at least one parameter includes at least one of: a spherical refractive power of the lens, a cylindrical refractive power of the lens, a cylinder axis of the lens, an asphericity value of the lens, a customized profile of the lens to compensate a higher order aberration of the eye.

3. The apparatus according to claim 1 or 2, wherein the means for determining are adapted to optimize a quantity representative of focus quality of rays (170) impinging on the retina (190).

4. The apparatus according to any of claims 1-3, wherein the apparatus further comprises means for obtaining at least one predetermined property of the lens (150) and the means for determining are adapted such as to optimize focusing on the retina (190) by ray tracing at least in part based on the at least one predetermined property.

5. The apparatus according to claim 4, wherein the at least one predetermined property of the lens (150) comprises at least one of: a position (140) of the lens, a refractive index of the lens, a thickness (155) of the lens, a type of the lens, a diameter of the lens, a profile of the lens.

6. The apparatus according to any of claims 1-5, wherein the topographic information of the anterior surface (110) and the topographic information of the posterior surface (120) of the cornea each comprises a plurality of data points spanning at least a surface area of 100 µm² on the anterior surface (110) as well as the posterior surface (120).

7. The apparatus according to any of claims 1-6, further comprising means for obtaining at least one parameter of an entrance pupil (P) of the eye (100); and wherein the means for determining are adapted such as to optimize focusing on the retina (190) by ray tracing at least in part based on the at least one parameter of the pupil (P) of the eye (100).

8. The apparatus according to any of claims 1-7, further comprising means for obtaining topographic information of a stromal interface (130) of the cornea, and/or an anterior chamber depth (140) of the eye (100); and
wherein the means for determining are adapted such as to optimize focusing on the retina (190) by ray tracing at least in part based on the topographic information of the stromal interface (130) of the cornea, and/or the anterior chamber depth (140) of the eye (100).

9. The apparatus according to any of claims 1-8, wherein the lens (150) is an intra-ocular lens to be implanted into the eye (100).

10. The apparatus according to any of claims 1-9, further comprising means for determining an optimized corneal anterior surface and/or a corneal ablation volume for correcting a residual aberration of the eye comprising the lens having the at least one parameter.

11. A computer-implemented method for determining at least one parameter of a lens (150) for an eye (100), the method comprising the following steps:
a. obtaining topographic information of an anterior surface (110) and topographic information of a posterior surface (120) of a cornea of the eye (100);
b. obtaining distance information (160) of a retina (190) of the eye (100) relative to the anterior surface (110) of the cornea of the eye (100);
c. determining the at least one parameter of the lens (150) of the eye (100) such as to optimize focusing on the retina (190) by ray tracing at least in part based on the topographic information of the anterior surface (110) of the cornea, the topographic information of the posterior surface (120) of the cornea and the distance information (160).

12. The method according to claim 11, wherein the at least one parameter includes at least one of: a spherical refractive power of the lens, a cylindrical refractive power of the lens, a cylinder axis of the lens, an asphericity value of the lens, a customized profile of the lens to compensate a high order aberration of the eye.

13. The method according to claim 11 or 12, wherein the determining includes optimizing a quantity representative of focus quality of rays (170) impinging on the retina (190).

14. The method according to any of claims 10-13, further comprising selecting an intraocular lens to be implanted into the eye at least in part based on the at least one determined parameter of the lens (150).

15. A computer program for determining at least one parameter of a lens (150) of an eye (100), the computer program comprising instructions which, when executed by a computer, cause the computer to implement the following steps:
a. obtaining topographic information of an anterior surface (110) and topographic information (120) of a posterior surface of a cornea of the eye (100);
b. obtaining distance information of a retina (190) of the eye relative to the anterior surface (110) of the cornea of the eye (100);
c. determining the at least one parameter of the lens (150) of the eye (100) such as to optimize focusing on the retina (190) by ray tracing at least in part based on the topographic information of the anterior surface (110) of the cornea, the topographic information (120) of the posterior surface of the cornea and the distance information (160).
